# EUROPEAN PATENT APPLICATION

(11) **EP 4 765 133 A1**
(43) Date of publication of application: **24.06.2026**
(21) Application number: 25224906.5
(22) Date of filing: 18.12.2025
(51) Int. Cl.: G16C 20/20, G16C 20/70, G16C 20/90

(54) **METHOD AND SYSTEM FOR COMPRESSION BASED SPECTRAL IDENTIFICATION**

(30) Priority: 20.12.2024 US 202463736652 P
(71) Applicant: Thermo Fisher Scientific Instruments LLC,, Madison, WI 53711 (US)
(72) Inventor: GLATZMAIER, Michael James, Maple Grove (US); HARRISON, Huimin Zeng, Madison (US); WILLE, Logan James, Madison (US)
(74) Representative: Boult Wade Tennant LLP

(57) **Abstract**

Systems or techniques are provided for spectral analysis. In various embodiments, a system can comprise a memory that stores computer executable instructions and a processor that executes the computer executable instructions. The computer executable instructions can comprise a compression component that compresses the one or more unknown spectra, using an encoder, into one or more compressed unknown spectra in a latent space; and a search component that matches the one or more compressed unknown spectra with one or more compressed known spectra to obtain one or more candidate matches of the sample.

## Description

### Field of the Invention

The present description relates generally to methods and systems for spectral data analysis, and more particularly, to determining sample composition based on the spectral data.

### Background

Spectral library searches are utilized to identify the material and/or chemical compositions corresponding to the unknown spectra. However, the size of such libraries makes scaling searches highly inefficient, both in terms of search time and storage costs.

### Summary

The following presents a summary to provide a basic understanding of one or more embodiments. This summary is not intended to identify key or critical elements, or delineate any scope of the particular embodiments or any scope of the claims. Its sole purpose is to present concepts in a simplified form as a prelude to the more detailed description that is presented later. In one or more embodiments described herein, devices, systems, computer-implemented methods, apparatus or computer program products that facilitate compression based spectral analysis and identification are provided.

According to one or more embodiments, a system is provided. The system can comprise a non-transitory computer-readable memory that can store computer-executable components. The system can further comprise a processor that can be operably coupled to the non-transitory computer-readable memory and that can execute the computer-executable instructions stored in the non-transitory computer-readable memory. In various embodiments, the computer-executable instructions can comprise a compression component that compresses the one or more unknown spectra acquire from a sample, using an encoder, into one or more compressed unknown spectra in the latent space, and a search component that matches the one or more compressed unknown spectra with one or more compressed known spectra to obtain candidate matches of the sample.

An advantage of the system, and/or of a corresponding computer-implemented method and/or computer program product can be improved speed at which the matching process can be performed and a reduction in computer resources required for such matching.

In one or more embodiments, the computer-executable instructions can further comprise a library look up component that retrieves one or more full spectra corresponding to the candidate matches and determines composition of the sample, based on a non-negative least squares comparison. The library look up component may retrieve the full spectra by decoding the compressed known spectra corresponding to the candidate matches using a decoder.

An advantage of the system, and/or of a corresponding computer-implemented method and/or computer program product can be the ability to operate without a local copy of a spectral library, thus reducing the storage requirements.

### Brief Description of the Drawings

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.
FIG. 1 is a block diagram of an example scientific instrument module for performing spectral analysis in accordance with various embodiments described herein.
FIG. 2 is a flow diagram of an example, non-limiting, method of performing spectral analysis in accordance with various embodiments described herein.
FIG. 3 and FIG. 4 illustrate block diagrams of example, non-limiting, scientific instruments that facilitate spectral analysis in accordance with one or more embodiments described herein.
FIG. 5 illustrates an example process of index generation in accordance with one or more embodiments described herein.
FIG. 6 illustrates an example method of single component search in accordance with one or more embodiments described herein.
FIG. 7 illustrates an example method of multicomponent search in accordance with one or more embodiments described herein.
FIG. 8 illustrates another example method of multicomponent search in accordance with one or more embodiments described herein.
FIG. 9 illustrates an example method of multicomponent multivariate search in accordance with one or more embodiments described herein.
FIG. 10 illustrates a chart comparing the performance of an existing spectra matching process to the compression-based approach as described herein.
FIG. 11 illustrates a flow diagram of an example, non-limiting, computer-implemented method that can facilitate unknown spectra matching in accordance with one or more embodiments described herein.
FIG. 12 illustrates a flow diagram of an example, non-limiting computer-implemented method that can train an encoder for spectra compression in accordance with one or more embodiments described herein.
FIG. 13 illustrates a block diagram of an example, non-limiting, operating environment in which one or more embodiments described herein can be facilitated.

### Detailed Description

The following detailed description is merely illustrative and is not intended to limit embodiments and/or application or utilization of embodiments. Furthermore, there is no intention to be bound by any expressed or implied information presented in the preceding Summary section, or in the Detailed Description section. One or more embodiments are now described with reference to the drawings, wherein like reference numerals are utilized to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

Spectral library searches are critical to identifying the chemical and/or material composition related to unknown spectra. As part of this process, unknown spectra acquired by a scientific instrument is compared to the known spectra contained within a spectral library. The known spectrum refers to spectrum of known chemical composition. The chemical composition includes one or more of the names of the chemical components and the proportions of the chemical components. The sample composition refers to the chemical composition of the sample. The spectral library contains known spectra of full resolution of various chemical components. A chemical component refers to an individual substance or compound that contributes to the overall composition of a sample being analyzed. Each chemical component may represent a distinct molecule, compound, or material phase that produces a characteristic spectrum measurable by analytical instruments such as FTIR, Raman, X-ray diffraction, or UV-Vis. In order to provide broad coverage of various potential chemical components, these spectra libraries often comprise thousands to millions of known spectra. The known spectra may be obtained from previous measurements and/or various spectra libraries. The spectra may be in the form of signal amplitudes at various wavelengths or wavenumbers. Different chemical composition corresponds to different spectral peaks. Each spectrum may include thousands of datapoints. This creates a problem when searching such libraries, as it induces large search times. Furthermore, the storage of such large libraries is an issue, preventing the local storage of such libraries on the computing devices or the scientific instruments.

To overcome the one or more deficiencies of existing technologies as identified above, one or more embodiments described herein can compress one or more unknown spectra acquired from a sample using an instrument, using an encoder, into one or more compressed unknown spectra in the latent space, and match the one or more compressed unknown spectra to one or more compressed known spectra, and obtain the candidate matches of the sample. The sample composition may then be decided based on the candidate matches. By operating directly in the latent space, this system significantly reduces the amount of data processed during matching, thereby enabling faster, more scalable spectral identification while reducing computing and storage demands.

The compression component may compress the unknown spectra by preprocessing the unknown spectra and passing the preprocessed spectra through the encoder, wherein the one or more unknown spectra may be preprocessed based on a configuration of the instrument and/or the encoder. The preprocessing may include one or more baseline correction, resampling, scaling, and smoothing. By preprocessing the unknown spectra to reduce noise, align amplitudes, and correct sampling inconsistencies, the system improves the consistency and accuracy of the encoded representation. Further, by preprocessing the unknow spectra, the same encoder may be used for different types of spectral data or spectral data acquired from different types of instruments. For example, the same encoder may be used for both benchtop and portable Raman or FTIR instruments. The preprocessing may be based on the configuration of the instrument, such as the imaging modality, the noise level and/or dimension of the acquired spectra. The preprocessing may also be based on the input and output requirements of the encoder. For example, the unknown spectra may be interpolated to meet the input dimension requirement of the encoder.

The compressed known spectra may be generated by compressing spectra of known chemical composition, using the encoder, into the compressed known spectra in the latent space. By encoding the known spectra in the same latent space as the unknown spectra, the system achieves reliable similarity comparison between latent representations. The compressed known spectra may be pre-computed and stored as a searchable index on a local device or in cloud storage.

The search component may obtain possible candidate matches by matching the one or more compressed unknown spectra with the one or more compressed known spectra, and select the one or more candidate matches from the possible candidate matches. A possible candidate match and a candidate match is the identifier, such as the name, of a chemical component, a specific sample, or a combination of multiple chemical components corresponding to a known spectrum (or a compressed known spectrum in the latent space). This two-stage search process first identifies a pool of possible matches based on similarity within the latent space and then refines the results to yield the most probable chemical candidates. The hierarchical approach reduces computational complexity and increases search precision across large datasets. Alternatively, the candidate matches can be directly selected, without obtaining the possible candidate matches.

Selecting candidate matches from the possible candidate matches may include clustering compressed known spectra corresponding to the possible matches. Clustering enhances robustness and diversity of candidate selection by reducing redundancy among similar spectra, leading to more reliable identification of chemical components. The selection of candidate matches may further comprise performing a regression comparison of the compressed unknown spectra with the compressed known spectra of the possible candidate matches. Regression filtering improves selection accuracy by quantifying the relationship between spectra of possible candidate matches and the unknown spectrum, prioritizing those contributing most significantly to the spectral profile.

Other methods, such as Nearest Neighbor-Based Ranking, Similarity Scoring and Threshold Filtering, Probabilistic or Density-Based Search, may alternatively be used to select the candidate matches from the possible candidate matches.

One or more of the compressed known spectra may be organized into an index. Herein, index refers to a search tree, which is a tree data structure containing the compressed known spectra. Examples of the search tree are k-d tree, ball-tree, r-tree, and cover tree. Each compressed known spectrum in the index corresponds to a pointer, which can be used to retrieve the component name or the full spectrum corresponding to the compressed known spectrum. Each compressed spectrum (compressed unknown and/or known spectrum) may be regarded as a latent space vector in the latent space. The term latent space vector refers to a compact numerical representation of spectral data produced by the encoder. When a spectrum containing a large number of data points, such as a full spectrum with intensity values across multiple wavelengths at high spectral resolution, is processed through the encoder, the encoder transforms this high-dimensional data into a lower-dimensional latent space. Each spectrum is thus represented as a latent space vector that captures the essential features of the original spectral signal. The latent space vector may have a predetermined dimension (elements or datapoints). The search tree may be formed by all compressed known spectra. The search tree may also be formed by a portion of the compressed known spectra. In other examples, instead of tree-based search structures, the search component may match the one or more compressed unknown spectra with the one or more compressed known spectra using hash-based, graph-based, partition-based, or AI-enhanced or learned index models.

In some examples, the compression component may further decompose the unknown spectra to generate component unknown spectra before compression. The decomposition step may increase the spectral search speed. The decomposition method may include multivariate curve resolution (MCR) methods.

In some examples, the search component may calculate one or more residual spectra and match the residual spectra with the compressed known spectra. Residual-based matching enables iterative refinement of multi-component identification, improving completeness of compositional determination. The search component may decide whether or not to calculate the residual spectrum based on prior knowledge of the unknown sample, such as the expected number of chemical components.

The residual spectra may be calculated by removing the compressed known spectra corresponding to the matched candidates from the compressed unknown spectra and performing subsequent matching of the residual spectra with the compressed known spectra. Alternatively, the residual spectra may be calculated by removing the full known spectra corresponding to the matched candidate from the un-compressed unknown spectra. The residual spectra may then be compressed by the encoder. Matching the residual spectra with the compressed known spectra includes matching the compressed residual spectra with the compressed known spectra.

A library lookup component may retrieve one or more full spectra corresponding to the candidate matches. The retrieved spectra may be obtained from remote servers or generated locally from compressed known spectra.

In one example, the library lookup component may retrieve the full spectra corresponding to the candidate matches by decoding the compressed known spectra using a decoder. When full-resolution spectra are required-such as for display, verification, or quantitative comparison-the system performs decompression or rehydration using a decoder. The decoder is trained to reverse the encoding process, reconstructing the full or near-original spectrum from the latent space vector. This rehydration process restores the spectral intensity patterns and peak characteristics that correspond to the chemical composition of the sample. When the encoder and decoder are jointly trained, the rehydrated spectrum maintains high fidelity to the original while still benefiting from the efficiency of latent-space storage and computation.

The library lookup component may determine the proportions of the candidate matches in the sample based on a non-negative least squares (NNLS) comparison between the full spectra and the unknown spectra. The NNLS computation ensures physically meaningful, non-negative estimates of component proportions, providing quantitative compositional analysis. Alternatively, the proportions of candidate matches may be determined using constrained least squares (CLS), Bayesian inference models, or iterative optimization algorithms. Machine learning regression models such as support vector regression or neural network predictors may also be used.

The system may include a training component configured to train the encoder. The encoder and decoder may be trained together with spectra of known chemical composition.

The training process may include augmenting a training dataset comprising spectra of known chemical compositions based on characteristics of the instrument, compressing and decompressing the training dataset using the encoder and the decoder, comparing the decompressed training data with the original data, and updating the encoder based on the comparison. This training process ensures encoder adaptability to instrument-specific variations and maintains high accuracy across different devices. Augmenting the spectra of known compositions based on instrument characteristics may include adding noise, scaling, shifting, and applying instrument-specific noise distributions.

The encoder designed for the first instrument may be used to spectral search for a second instrument. The first and second instruments may have different configurations, such as hardware configurations. For example, the first instrument may be a benchtop Raman system and the second instrument a handheld, portable Raman system. The instruments may have different detectors. The encoder trained for the first system may be directly used for the second system. This may allow interoperability of the encoder across instruments with varying configurations, reducing the need for separate calibration or retraining. Applicants also noticed that the first and second instruments can be of different imaging modalities. For example, the first instrument may be for FTIR imaging, and the second instrument is for Raman imaging. As such, the training data may include spectra of different types. The encoder for a Raman system can be trained with both FTIR and Raman spectra.

One or more embodiments are now described with reference to the drawings, where like referenced numerals are used to refer to like elements throughout. In the following description, for purposes of explanation, numerous specific details are set forth to provide a more thorough understanding of the one or more embodiments. It is evident in various cases, however, that the one or more embodiments can be practiced without these specific details.

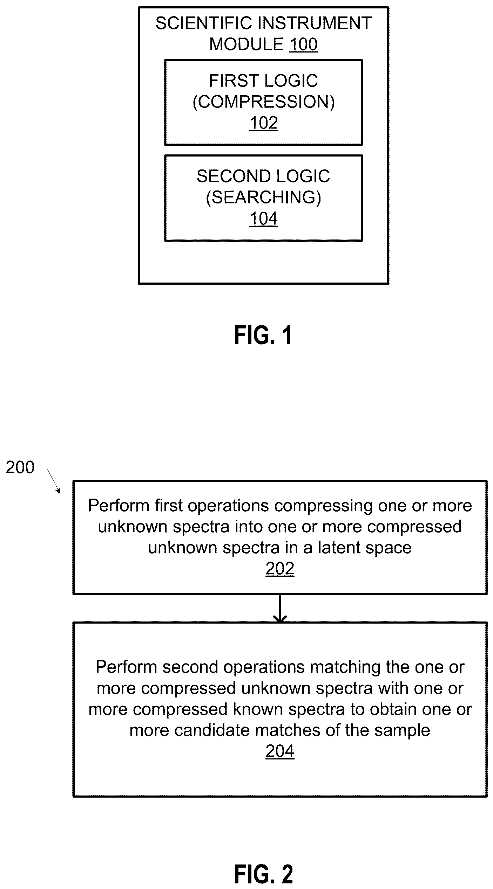

FIG. 1 illustrates an example, non-limiting block diagram of a scientific instrument module 100 in accordance with various embodiments described herein.

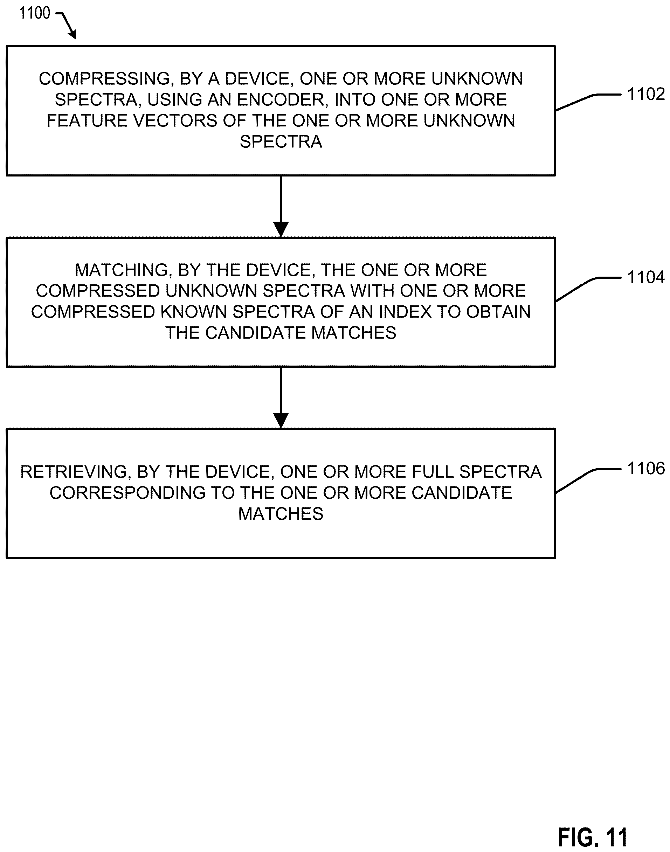

In various embodiments, the scientific instrument module 100 can be implemented by circuitry (e.g., including electrical or optical components), such as a programmed computing device. Logic of the scientific instrument module 100 can be included in a single computing device or can be distributed across multiple computing devices that are in communication with each other as appropriate. Examples of computing devices that may, singly or in combination, implement the scientific instrument module 100 are discussed herein with reference to FIG. 11.

The scientific instrument module 100 may include first logic 102 and second logic 104. As used herein, the term "logic" may include an apparatus that is to perform a set of operations associated with the logic elements. For example, any of the logic elements included in the scientific instrument module 100 may be implemented by one or more computing devices programmed with instructions to cause one or more processing devices of the computing devices to perform the associated set of operations. In a particular embodiment, a logic element may include one or more non-transitory computer-readable media having instructions thereon that, when executed by one or more processing devices of one or more computing devices, cause the one or more computing devices to perform the associated set of operations. As used herein, the term "module" may refer to a collection of one or more logic elements that, together, perform a function associated with the module. Different ones of the logic elements in a module may take the same form or may take different forms. For example, some logic in a module may be implemented by a programmed general-purpose processing device, while other logic in a module may be implemented by an application-specific integrated circuit (ASIC). In another example, different ones of the logic elements in a module may be associated with different sets of instructions executed by one or more processing devices. A module may not include all of the logic elements depicted in the associated drawing; for example, a module may include a subset of the logic elements depicted in the associated drawing when that module is to perform a subset of the operations discussed herein with reference to that module.

In various embodiments, there can be a scientific instrument corresponding to the scientific instrument module 100. In various aspects, the scientific instrument can be any suitable computerized device that can electronically measure some scientifically-relevant, clinically-relevant, or research-relevant characteristic, property, or attribute of an analytical sample (e.g., of a known or unknown mixture, compound, or collection of matter). As a non-limiting example, a scientific instrument can be a mass spectrometer. In such a case, the scientific instrument can measure or determine ion spectra (e.g., relative ion abundance as a function of mass-to-charge ratio) of the analytical sample.

In some examples, the scientific instrument is an analytical instrument that acquires spectral data from a sample. The scientific instrument may include a source for emitting radiations, such as electromagnetic waves, to the sample and a detector for acquiring signals generated from the sample responsive to the radiation. The detector may be a spectrograph, and the acquired signals are in the form of spectra. The scientific instrument may be one of a Raman microscopy system, a FT-IR microscopy system, a UV-Vis spectroscopy system, an X-ray spectroscopy system, a mass spectrometry system, and an X-ray fluorescence system. In some examples, the scientific instrument is portable or handheld. The scientific instrument may include a display for displaying the data including the spectra to the operator.

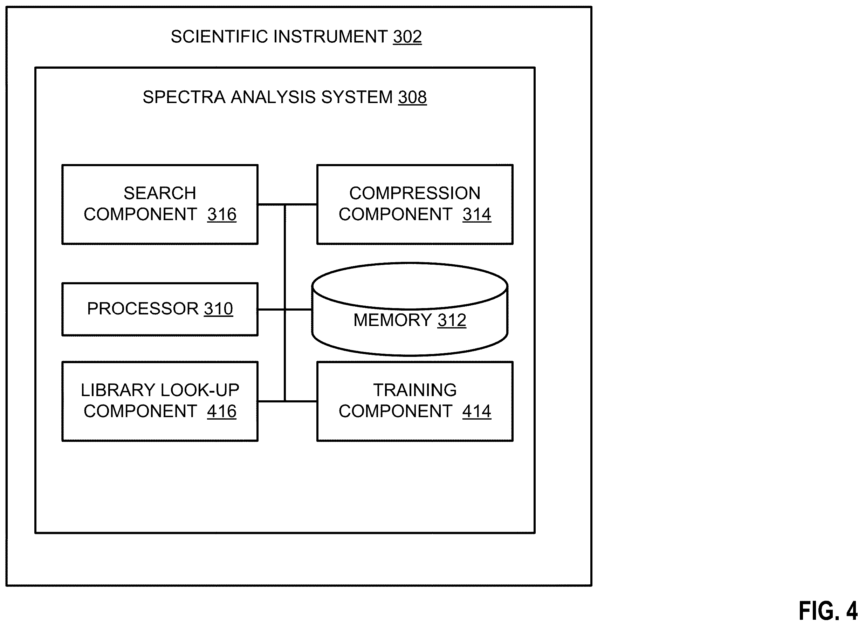

The first logic 102 may compress one or more spectra. The spectra may be unknown spectra acquired by the instrument or a different instrument from a sample with unknown composition. The spectra are compressed by an encoder. In one example, the unknown spectrum is preprocessed before being compressed by the encoder, to match the input parameters or requirements of an encoder. The preprocessing may include one or more of baseline correction, re-sampling, scaling and smoothing. The preprocessed unknown spectra are then sent through the encoder including one or more layers to produce a latent space representation of the unknown spectra, alternatively referred to as one or more latent space vectors or compressed spectra. The encoder may be pretrained or trained using a training dataset, as described in relation to in FIG. 4.

The second logic 104 may match the one or more compressed spectra (e.g., latent space vectors) to one or more compressed known spectra and obtain candidate matches from an index of the compressed known spectra. For example, a search of a search tree generated from the compressed known spectra is first performed to generate the possible candidate matches. The candidate matches are then selected from the possible candidate matches. The search may be the nearest neighbor search, cosine similarity search, or support vector machine. The first logic 102 and the second logic 104 may work interactively together to perform multiple types of searches to identify one or more chemical components corresponding to the unknown spectra.

FIG. 2 is a flow diagram of a computer-implemented method 200 in accordance with one or more embodiments described herein. The operations of the computer-implemented method 200 may be used in any suitable setting to perform any suitable operations described herein. Operations are illustrated once each and in a particular order in FIG. 2, but the operations may be reordered or repeated as desired and appropriate (e.g., different operations performed may be performed in parallel, as suitable).

At 202, first operations may be performed. For example, the first logic 102 of scientific instrument module 100 may perform the operations of 202. The first operations may include compressing one or more unknown spectra into one or more compressed unknown spectra in a latent space. Compressing the spectra may include preprocessing the spectra to a format expected by an encoder.

At 204, second operations may be performed. For example, the second logic 104 of scientific instrument module 100 may perform the operations of 204. The second operations may comprise matching the one or more compressed unknown spectra with one or more compressed known spectra to obtain one or more candidate matches of the sample.

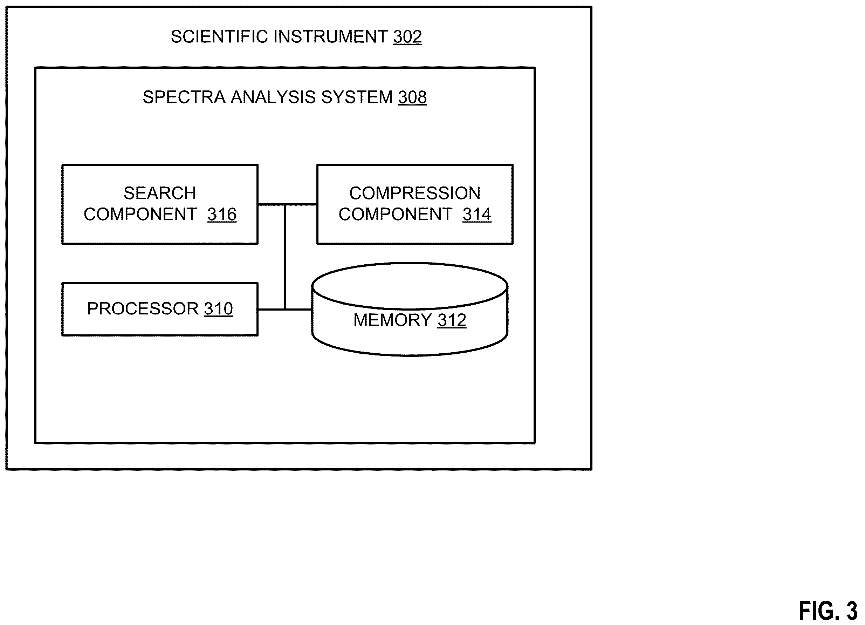

FIG. 3 illustrates a block diagram of an example, non-limiting scientific instrument that can facilitate spectra analysis in accordance with one or more embodiments described herein.

In various embodiments, the scientific instrument 302 can comprise a spectra analysis system 308. In various cases the spectra analysis system 308 can analyze the unknown spectra and determine the chemical components (candidate matches) contributed to the spectra. The spectra analysis system 308 may also determine the proportions of the chemical components in the sample.

In various aspects, the spectra analysis system 308 comprises a processor 310 (e.g., computer processing unit, microprocessor) and a non-transitory computer-readable memory 312 that is operably or operatively or communicatively connected or coupled to the processor 310. The non-transitory computer-readable memory 312 can store computer-executable instructions which, upon execution by the processor 310, can cause the processor 310 or other components of the spectra analysis system 308 (e.g., compression component 314 and search component 316) to perform one or more acts. In various embodiments, the non-transitory computer-readable memory 312 can store computer-executable components (e.g., compression component 314 and search component 316), and the processor 310 can execute the computer-executable components.

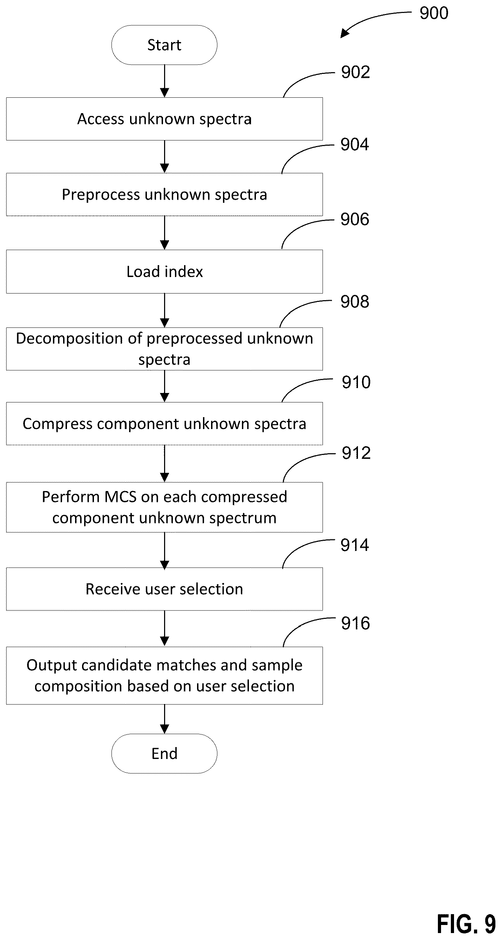

The spectra analysis system 308 comprises a compression component 314. In one or more embodiments, compression component 314 can preprocess and then compress, using an encoder or another suitable compression technique, one or more spectra. For example, the preprocessing can comprise preparing the spectra to match the input parameters of the encoder. The preprocessing can comprise any combination of resampling the unknown spectra to fit an input size, scaling, baseline correction, and a smoothing process to reduce noise in the spectra. Resampling a spectrum can include sub-sampling, interpolating, or padding the spectrum to meet a predetermined number of datapoints (element size). Resampling may also include normalizing and/or cropping the spectrum to a predetermined amplitude range. The baseline correction process may isolate and remove background signals, for example caused by noise or scattering, from chemically relevant signals. Baseline correction may remove baseline noises caused by the spectral acquisition system or the spectral acquisition environment. Baseline correction may also include removing background noise such as the fluorescence background in Raman signal acquisition or background noise in FTIR signal acquisition. Smoothing includes filtering the spectrum. It should be appreciated that use of additional preprocessing steps is envisioned. In one or more embodiments, the unknown spectra can be collected and/or generated using an instrument, such as a mass spectrometer, a Raman spectrometer, a FTIR spectrometer, communicatively coupled with spectra analysis system 308. In some embodiments, the spectra analysis system 308 is a part of the instrument that acquires the unknown spectra from a sample. The compression component 314 may additionally decompose the unknown spectra into multiple component unknown spectra (as shown in FIG. 9) after preprocessing the unknown spectra.

Compression component 314 compresses the spectra through an encoder and generate the compressed spectra. The encoder encodes the spectra into latent space vectors in the latent space. The unknown spectra can be compressed by the encoder into compressed unknown spectra, and the known spectra can be compressed by the same encoder into compressed known spectra. The compression component may further arrange the compressed known spectra into an index. The index may be stored in the memory and loaded when performing the component search.

The encoder is trained using training dataset including the known spectra, and is stored in the memory 312 of the spectral analysis system 308. For example, the encoder may be set to accept inputs of an unknown spectrum including 3000 elements (or datapoints) and generate a latent space vector of 200 elements (or datapoints). In this manner, the size of the unknown spectra can be compressed, while still retaining the relevant information in order to enable an effective match of the unknown spectra. It should be appreciated that use of any number of input elements or the size of latent space representations (number of latent space vectors) is envisioned.

The encoding can be achieved for example by an AI enhanced autoencoder or randomized projection matrices. The AI enhanced autoencoder may be any one or more of 1D convolutional autoencoders, deep feedforward autoencoders, variational autoencoders, and recurrent autoencoders. In randomized projection matrices, compression is achieved by performing the spectral dot produce with the library spectra and a basis set of randomly generated projection matrices. These random projections form a low-dimensional feature set for each library spectra, allowing for configurable levels of data compression. It should be appreciated that the use of other machine learning methods that produce latent space representations, such as variational encoders or recurrent networks, is envisioned.

In various embodiments, search component 316 matches the one or more compressed unknown spectra with the compressed known spectra to obtain one or more candidate matches. The search component may further determine the composition corresponding to the known spectra.

The search component 316 can build an index from the compressed known spectra. For example, search component 316 can access the compressed known spectra generated by the compression component or in the cloud. An index, such as a k-distance (k-d) tree, can then be generated from the compressed known spectra wherein similar compressed known spectra are stored close to one and other in the tree structure. In some examples, search component 316 can access multiple libraires and build a single or multiple indexes from the known spectra in the libraries.

The search component 316 can match the one or more compressed unknown spectra with the compressed known spectra in the index to obtain the candidate matches. The search component can further determine sample composition based on the candidate matches.

The compression component 314 and the search component 316 can work together to achieve various types of searches including single component search (SCS), multicomponent search (MCS), and multivariate multicomponent search (MMCS) for determining sample composition of the unknown spectra. The spectra analysis system 308 may output sample composition. The spectra analysis system 308 may also display the composition information and the associated spectral data to the user.

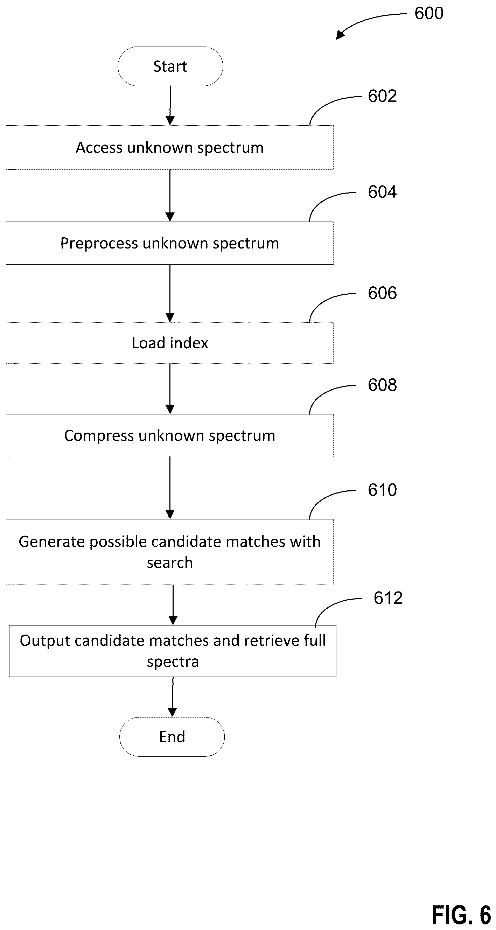

In the SCS, a single known spectrum is matched to a single unknown spectrum. The sample of the unknown spectrum contains one chemical component, therefore one candidate match. In some examples, the SCS may output multiple candidate matches, wherein the compressed known spectra of the candidate matches have a high similarity (similarity higher than a predetermined threshold similarity) to the compressed unknown spectrum. Details of the SCS mode are shown in FIG. 6.

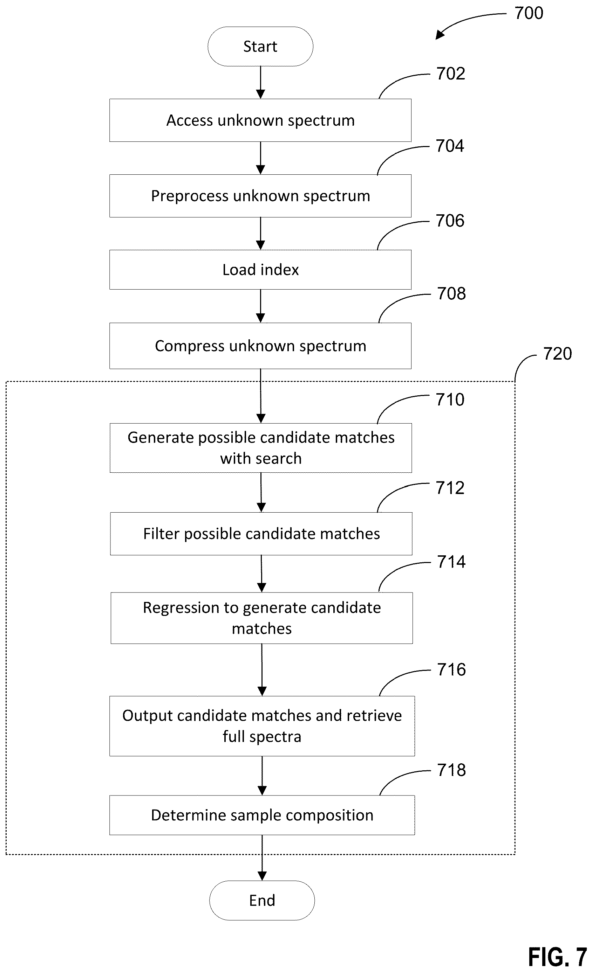

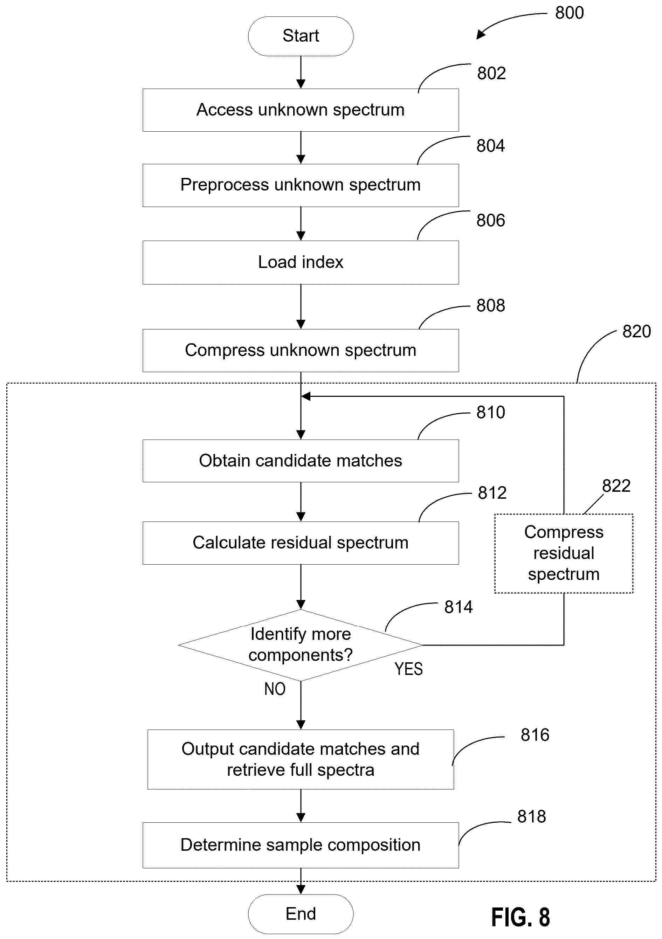

In the MCS, multiple known spectra are matched to a single known spectrum. The sample of the unknown spectrum contains multiple chemical components. The MCS outputs multiple candidate matches. Further, sample composition can be determined based on the contribution of each candidate match to the unknown spectrum. Details of the MCS are presented in FIG. 7 and FIG. 8.

In the MMCS, multiple known spectra are matched or mapped to a series of unknown spectra. The series of unknown spectra may be acquired from a sample over a duration of time or over a spatial range. In one example, series of unknown spectra are acquired over time while the sample is undergone a chemical reaction. Each of the unknown spectra is acquired at different time point. In another example, each of the unknown spectra is acquired from different locations of a sample. MMCS can also determine sample composition. Details of the MMCS are presented in FIG. 9.

FIG. 4 illustrates a block diagram of an example, non-limiting scientific instrument that can facilitate spectral analysis in accordance with one or more embodiments described herein. As shown, scientific instrument 302 can comprise spectra analysis system 308 as described above in relation to FIG. 3. Spectra analysis system 308 of FIG. 4 can further comprise library look-up component 416 and/or training component 414.

In various embodiments, library look-up component 416 can retrieve one or more full spectra corresponding to the candidate matches. The full spectrum may have the same resolution as the uncompressed known spectrum in the spectral library. The full spectra may be the uncompressed known spectra in the spectral library. Alternatively, the full spectra may be reconstructed from the compressed known spectra. The full spectra corresponding to the candidate matches can be retrieved from the known spectra in spectral library by the library look-up component 416 with pointers in the search tree. In one or more embodiments, the spectral library can be stored remotely, such as on a server, allowing retrieval over a network and eliminating the need for the library to be stored locally. This can greatly reduce the memory and storage requirements of devices performing the matching operations, as the spectral library can require hundreds of gigabytes of storage. In another sample, the library look-up component 416 includes a decoder that enables decompression, or rehydration, the compressed known spectra. The decoder may be generated while training the encoder with the training dataset. For example, the decoder can work in reverse of the encoder, enabling recovery of the full spectra from the compressed version. Accordingly, in this rehydrating process, the library look-up component 416 can retrieve the full spectra by passing the compressed known spectra through the decoder. This can allow for retrieval of the uncompressed spectra without the need to connect to the spectral library across the network.

In various embodiments, the training component 414 can train or retrain the encoder and decoder with the training dataset. In one or more embodiments, the training data set compresses known spectra, such as a portion of or the complete spectral library.

Training component 414 may optionally augment the training data based on the characteristics of the instrument for acquiring the unknown spectral data. For example, training data augmentation may be required for known spectra obtained from public databases. These instrument characteristics may be stored in the memory 312. These characteristics include one or more of the noise level, baseline, spectral shift, spectrum peak of the acquired spectral data, and the instrument specific noise distribution. The unique characteristics of the instrument are determined by the configuration of the instrument, as well as the specifications of the system components, such as the specifications of the camera and spectrograph. In one example, the training data set may be augmented by adding instrument specific noise (e.g., Poisson noise), scaling, introducing x-shifts, and broadening the peaks. Furthermore, training component 414 can add specific noise distributions that are likely to occur in data collected from specific instrument types. Additionally, spectral broadening can be performed by fitting various distributions (e.g., Voigt distributions) to the training data and then adjusting a width parameter of the distribution to simulate instrumentation broadening. The augmentation process densely samples the possible instrument specific signatures from a variety of instrument and laboratory settings. This improves the robustness of the training process and reduces the sensitivity of the encoder to instrument specific effects. The training data can then be preprocessed in the same manner as will be utilized for the index generation and the compression of unknown spectra.

During the training process, the training data can be compressed by the encoder and decompressed by the decoder. The decompressed output can then be compared to the training data as input and an objective function can be utilized to update the layers of encoder and decoder to attempt to maximize the similarity between the input training data and the decompressed output. In this manner training component 414 can train the encoder and decoder to generate accurate compressed representation of spectra.

In some examples, the trained encoder and decoder can be retrained with new training dataset. The new training dataset may be acquired by a user using the scientific instrument or a different instrument or system, from samples with known composition. The user may repeat the training process on the new training dataset to obtain the retrained encoder and decoder. The new training dataset may be preprocessed as described above in relation to FIG. 3, before inputting to the encoder. The retrained encoder and decoder may be stored in the scientific instrument for spectral data compression and search. The data augmentation process may be omitted if the new training dataset is acquired from the same or similar system that was used to acquire the unknown spectral data.

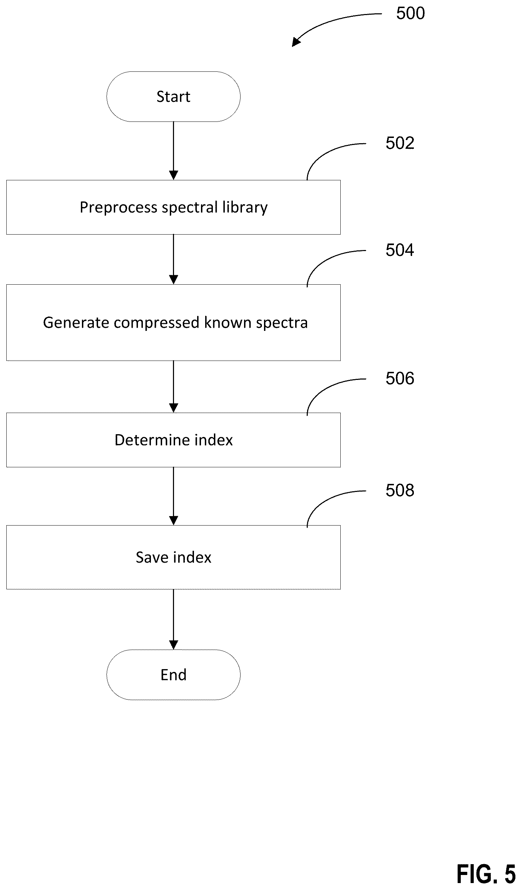

FIG. 5 illustrates an example method 500 for generating the index from the spectral libraries in accordance with one or more embodiments described herein.

At 502, the spectral library that including spectra of known chemical composition (i.e., known spectra) are preprocessed. For example, as described above in relation to FIG. 3, compression component 314 can preprocess the spectra within the spectral library by resampling, scaling and smoothing the spectra to make the spectra suitable to input for the encoder.

At 504, the compressed known spectra (or latent space vectors) are generated. For example, as described above in relation to FIG. 3, compression component 314 can compress each of the preprocessed known spectra from 502 by passing each spectrum through a trained encoder and obtaining the latent space vector. In this manner, the spectra can be compressed with minimal loss of the information relevant to making accurate matches.

At 506, the index can be determined by building a search tree from the compressed known spectra. For example, as described above in relation to FIG. 3, the compressed known spectra can be stored in a search tree (such as k-d tree) based on similarity to one another.

At 508, the index is saved for future matching/searching operations. Due to the compression performed on the known spectra, the overall storage size of the index is greatly reduced in comparison to the spectral library of the known spectra, thereby enabling storage on devices with more limited storage capabilities.

In some examples, the index is built and stored in a different system that compressed the known spectra.

In some examples, only the compressed known spectra are saved, and the index can be generated during the matching/searching process in the same or a different system that compressed the known spectra.

FIG. 6 illustrates an example method 600 of determining the chemical component that corresponds to the unknown spectra in accordance with one or more embodiments described herein. Method 600 corresponds to the SCS process wherein each known spectrum is matched to one unknown spectrum.

At 602, method 600 accesses the unknown spectrum. The unknown spectrum may be one of multiple unknown spectra acquired by an instrument.

At 604, the unknown spectrum is preprocessed. For example, as described above in relation to FIG. 3 and FIG. 4, compression component 314 can preprocess the unknown spectrum by resampling, scaling and smoothing the spectrum to make the spectrum suitable input for the encoder.

At 606, the index generated from method 500 is loaded for use in the matching operation. In some examples, if no index is available, the index can be generated based on the compressed known spectra as described in 506 of method 500. In some examples, multiple indexes are loaded.

At 608, the preprocessed unknown spectrum from 604 is compressed to generate the compressed unknown spectrum. For example, as described above in relation to FIG. 3 and FIG. 4, compression component 314 generates latent space vector from preprocessed unknown spectrum by passing the spectrum through a trained encoder. The trained encoder may be the same encoder used in 504 of FIG. 5.

At 610, the compressed unknown spectrum from 608 is matched with the compressed known spectra in the index to obtain the possible candidate matches. As described above in relation to FIG. 3 and FIG. 4, the search component 316 can perform a recursive nearest neighbor search (such as a FLANN search) that identifies neighbors (e.g., compressed known spectra) of the compressed unknown spectra from 608 based on a similarity metric comparison, such as cosine similarity. Accordingly, all compressed known spectra within a pre-defined similarity/distance of the unknown compressed spectra can be identified, and the possible candidate matches corresponding to the identified compressed known spectra are obtained (for example via the pointer to the identified compressed known spectra). The pre-defined similarity/distance may be a similarity higher than a threshold similarity or a distance smaller than a threshold distance. In some examples, a predefined number of possible candidate matches with higher similarity are output. For example, N possible candidate matches are identified based on the nearest N neighbors identified, wherein N is a number specified by the user.

At 612, method 600 outputs the candidate matches selected from the possible candidate matches. In one example, all possible candidate matches are output as candidate matches. The candidate matches can be displayed to a user in the form of a sorted list. The candidate matches in the list may be sorted based on the similarity metric comparison of 610. In some examples, the full spectra corresponding to the candidate matches can be retrieved and optionally displayed. As presented in FIG. 4, the full spectra may be retrieved from the spectral library, or alternatively, generated from the corresponding compressed known spectra through the rehydrating process.

FIG. 7 illustrates an example method 700 for determining chemical composition of an unknown spectrum, in accordance with one or more embodiments described herein. Method 700 corresponds to the MCS process that matches multiple known spectra to each unknown spectrum to determine multiple chemical components contributing to the unknown spectrum.

At 702, 704, 706, and 708, similar to steps 602, 604, 606, and 608 of FIG. 6, the unknown spectrum is accessed, preprocessed, and compressed into compressed unknown spectrum. One or more of the indexes are loaded. If there is no index available, step 708 may include generating the index from the compressed known spectra, as described in 506 of FIG. 5.

At 710, similar to 610 of FIG. 6, the compressed unknown spectrum is matched to the compressed known spectra in the index using a recursive nearest neighbor search, and possible candidate matches are generated. The number of the possible candidate matches may be predetermined. For example, the number of the possible candidate matches is 1000.

At 712 and 714, candidate matches are selected from the possible candidate matches. At 712, the number of the possible candidate matches are reduced based on clustering of the known spectra of the possible candidate matches. The possible candidate matches are filtered/reduced by selecting the compressed known spectra of the possible candidate matches which correlate with the compressed unknown spectra but are dissimilar to one and other. In other words, among the possible candidate matches, the ones with compressed known spectra that correlate well with the compressed unknown spectra but are diverse in their properties are selected. In one example, the filtering may include a maximized margin filtering that rejects spectra that have a cosine similarity below a defined threshold. A clustering operation is performed on the remaining spectra, such as kMeans clustering, to classify the remaining spectra into one or more clusters and then a pre-defined number of remaining spectra can be selected from each cluster using a cosine similarity to remove highly similar candidates. For example, the pre-defined number of remaining spectra is 100.

At 714, regression is performed on the compressed known spectra corresponding to the filtered possible candidate matches to further select the candidate matches from the possible candidate matches. By performing the regression, a pre-defined number of candidate matches are determined. For example, search component 316 can execute a regression, such as a LASSO (least absolute shrinkage and selection operator) regression, on the compressed known spectra of the filtered possible candidate matches to generate possible combinations of the compressed known spectra with the largest LASSO coefficients, favoring sparse solutions. For example, pre-defined number of candidate matches is 10. The regression encourages sparsity in the final linear combination (step 718) and can efficiently and robustly return the final selection of candidate matches.

At 716, method 700 outputs candidate matches. The full spectra corresponding to the candidate matches may also be retrieved as presented in FIG. 4.

At 718, the sample composition, such as the proportion of each candidate matches, may be optionally determined based on the retrieved full spectra of the candidate matches. A linear combination of retrieved full spectra is determined to match the unknown spectrum. For example, search component 316 performs a non-negative least squares operation to estimate the relative contributions of the retrieved full spectra that best matches the unknown spectrum. The non-negative least squares operation ensures that the fitting coefficients (corresponding to the proportions of the candidate match) are positive.

The core procedure 720 of the MCS includes steps 710, 712, 714, 716, and 718, wherein the candidate matches contributing to the unknown spectrum are determined, and the sample composition is calculated.

FIG. 8 illustrates another example method 800 of MCS in accordance with one or more embodiments described herein. Similar to method 700 of FIG. 7, method 800 matches multiple known spectra to one unknown spectrum and determine the multiple chemical components contributing to the unknown spectrum. Different from method 700, method 800 includes an iteration process in its core procedure 820 (810 - 818) to identify the candidate matches sequentially.

At 802, 804, 806, and 808, similar to steps 602, 604, 606, and 608 of FIG. 6, the unknown spectrum is accessed, preprocessed, and compressed. One or more of the indexes are loaded. If the index of compressed known spectra has not been built, the index is generated from the loaded compressed known spectra.

At 810, the candidate matches are identified for the compressed spectrum. If no residual spectrum has been calculated, the compressed spectrum is the compressed unknown spectrum from 808. Otherwise, the compressed spectrum is the residual spectrum calculated from 812. For example, multiple candidate matches of compressed spectrum can be obtained similar to 610 and 612 of FIG. 6, via recursive nearest neighbor search. In some examples, one candidate match (that is, one chemical component) that has the highest similarity score is identified.

At 812, the residual spectrum may be calculated by subtracting the one or more compressed known spectra of the candidate matches obtained from 810 from the compressed spectrum from 808. The residual spectrum may alternatively be calculated by subtracting the full known spectra of the candidate matches obtained from 810 from the unknown spectrum from 804.

Step 812 may include scaling one or more of the compressed spectra before subtraction. In some examples, the proportion/composition of the candidate matches can be determined based on the scaling factors.

At 814, method 800 determines whether further sample component need to be identified. In one example, more components need to be identified if the signal strength of the residual spectrum exceeding a predetermined threshold. In another example, more components need to be identified if the difference of between the current residual spectrum and the previous residual spectrum exceeds a predetermined threshold. In yet another example, the number of the iterations is determined based on the number of components (number of candidate matches) pre-selected by the user.

If the answer at 814 is YES, method 800 moves to 810 and more components or candidate matches are determined. If the residual spectrum is calculated by subtracting the full known spectra of the candidate matches from the unknown spectrum, the residual spectrum is further compressed at 822, by the encoder, before obtaining further candidate matches at 810.

If the answer at 814 is NO, method 800 moves to 816.

At 816, all candidate matches obtained at 810 are output or stored. Full spectra are retrieved. At 818, similar to 718 of FIG. 7, proportions of the various candidate matches can be calculated via the non-negative least squares operation.

FIG. 9 illustrates an example method 900 MMCS in accordance with one or more embodiments described herein. In MMCS, multiple known spectra are matched to multiple unknown spectra to determine multiple chemical components contributing to the unknown spectra. The multiple unknown spectra may be spatial or temporal related and acquired from the same sample.

At 902, 904, and 906, similar to steps 602, 604, and 606 of FIG. 6, the unknown spectra are accessed and preprocessed. One or more of the indexes are loaded. If the index has not been built, the index is generated from the loaded compressed known spectra as presented in 506 of FIG. 5.

At 908, decomposition is performed on the unknown spectra to generate component unknown spectra. The component unknown spectra are statistical independent from each other. For example, the decomposition may be implemented using a multivariate curve resolution (e.g., MCR) method.

At 910, the component unknown spectra are compressed into compressed unknown spectra. As described above in relation to FIG. 3 and FIG. 4, compression component 314 can preprocess the component unknown spectra and generate the compressed unknown spectra using the encoder.

At 912, MCS procedure is performed on each of the compressed unknown spectra to identify the candidate matches. The MCS procedure may be either the core procedure 720 of FIG. 7 or the core procedure 820 of FIG. 8. Since multiple MCS is performed on the spectral dataset, the predefined number of possible candidate matches and the predefined number candidate matches may be decreased with the increased number of unknown spectra.

At 914, method 900 receives user selection of displaying specific sample and spectral information. For example, the user may select one or more time slices or time ranges. In another example, the user may select one or more portions of the sample.

At 916, method 900 outputs the candidate matches and the sample composition, based on the user selection. For example, the chemical components at the user selected temporal or spatial region may be displayed. The proportion of the chemical components at different selected temporal or spatial regions may also be displayed. The candidate matches and the sample composition of the series of unknown spectra may be stored.

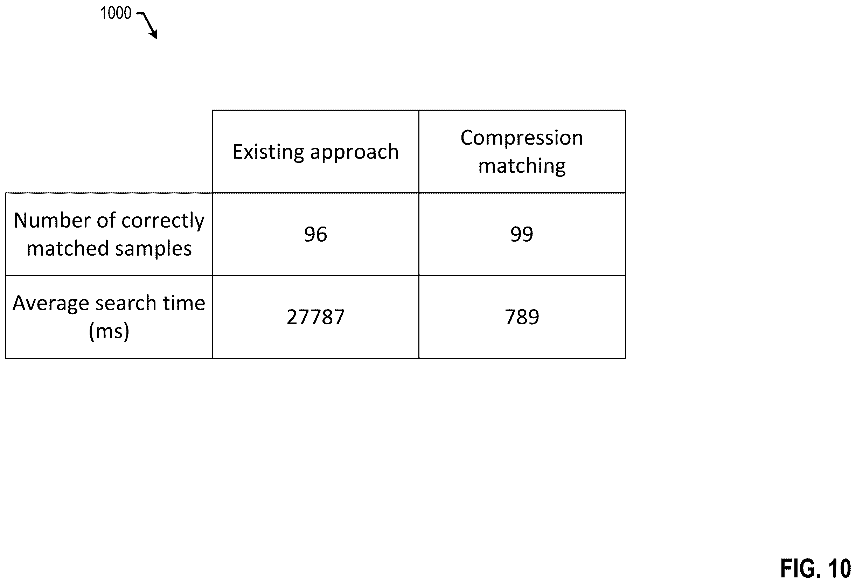

FIG. 10 illustrates a chart 1000 comparing the performance of an existing spectra matching process to the compression-based approach as described herein.

For chart 1000, 300 MCS were performed using an existing approach and the compression matching described herein. As shown, the compression approach offered slightly superior accuracy while executing approximately 35 times faster. Accordingly, the approach described herein can enable spectra search in decreased amounts of time. Furthermore, this enables the search and matching to be performed on devices with lower computational capabilities. Furthermore, the compressed spectral library in this experiment was three times smaller than the full spectra library, thus decreasing the amount of storage and/or memory required to execute search and match operations.

FIG. 11 illustrates a flow diagram of an example, non-limiting, computer-implemented method 1000 that can facilitate unknown spectra matching in accordance with one or more embodiments described herein.

In various cases, spectra analysis system 308 can facilitate the computer-implemented method 1100. In various embodiments, act 1102 can comprise compressing, by a device (e.g., compression component 314), one or more unknown spectra into one or more latent space vectors of the one or more unknown spectra. For example, as described above in reference to FIG. 3 to FIG. 9, compression component 314 can resample, scale and smooth the one or more unknown spectra and then pass the unknown spectra through an encoder to generate a compressed latent space representation of the one or more unknown spectra.

In various embodiments, act 1104 can comprise matching, by the device (e.g., search component 316), the one or more compressed unknown spectra with one or more compressed known spectra of an index to obtain the candidate matches. For example, as described above in reference to FIG. 3 to FIG. 9, search component 316 can perform a nearest neighbor search on the index that returns possible candidate matches.

In various embodiments, act 1106 can comprise retrieving, by the device (e.g., library look-up component 416) one or more full spectra corresponding to the candidate matches. For example, library look-up component 416 can retrieve uncompressed versions of the one or more components from a spectral library. In another embodiment, library look-up component 416 can retrieve the full spectra by rehydrating the compressed known spectra with a decoder corresponding to the encoder.

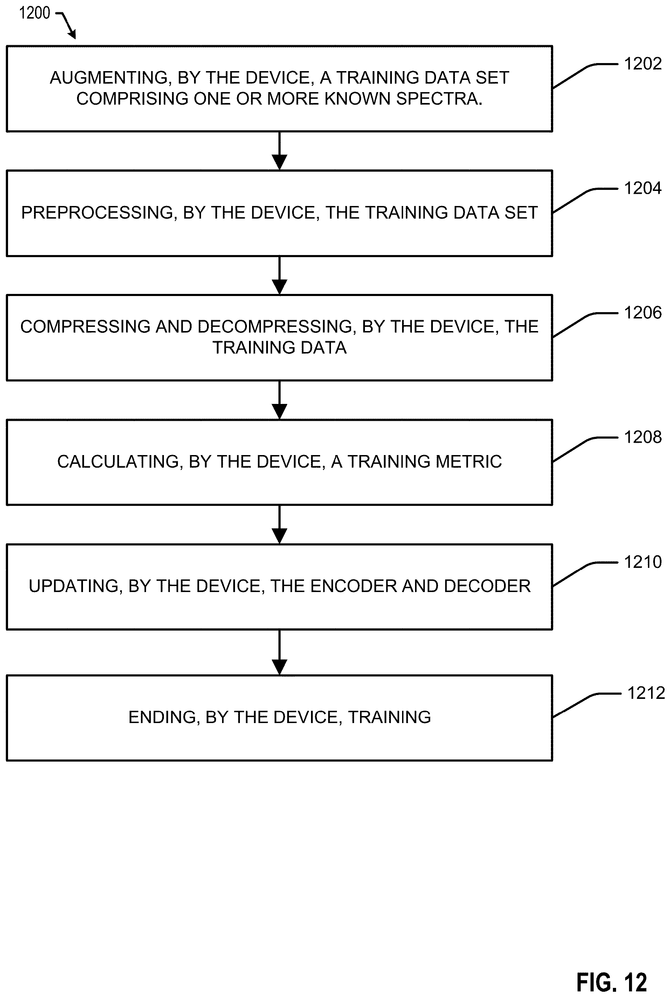

FIG. 12 illustrates a flow diagram of an example, non-limiting computer-implemented method 1200 that trains an encoder and the decoder for spectra compression in accordance with one or more embodiments described herein.

In various cases, spectra analysis system 308 can facilitate the computer-implemented method 1200. In various embodiments, act 1202 can comprise, augmenting, by a device (e.g., training component 414), a training data set comprising one or more known spectra. For example, as described above in relation to FIG. 3 to FIG. 4, the training dataset can comprise part or all of a spectral library. The spectral library may include spectra of known chemical composition. The known spectra may be public available spectra or spectra acquired from various instruments. Furthermore, training component 414 can augment the training data based on characteristics of the instrument for acquiring the unknown spectra data.

In various embodiments, act 1204 can comprise preprocessing, by the device (e.g., compression component 314) the training data set. For example, as described above in relation to FIG. 3 to FIG. 4, the training dataset can be preprocessed in the same manner that unknown spectra will be during operations.

In various embodiments, act 1206 can comprise compressing and decompressing, by the device (e.g., compression component 314) the training data set. For example, the spectra in the training data set can be compressed using the encoder and then decompressed using the decoder.

In various embodiments, act 1208 can comprise calculating, by the device, a training metric. The training metric can be a loss function or a difference between the uncompressed and the training data. For example, the difference is a cosine similarity between the uncompressed training data and a copy of the training data after preprocessing, but before compression.

In various embodiments, act 1210 can comprise updating, by the device, (e.g., training component 414), the encoder and decoder based on the comparison. For example, training component 414 can adjust the parameters of the encoder and/or decoder in order to increase similarity between uncompressed training data and the stored copy of the preprocessed training data. Act 1208 and 1210 may be repeated till a predefined criteria is met. For example, the parameters of the encoder and/decoder are adjusted till a stagnation of the training metric is met, such as the change of the metric between iteration is less than a threshold of change.

At act 1212, the training of the encoder and the decoder are completed. The trained encoder and decoder may be stored in the memory (such as memory 312 of FIG. 3 and FIG. 4) for compressing and rehydrating the unknown spectra.

An advantage of the systems, and/or of corresponding computer-implemented methods and/or computer program products described herein can be the ability to more accurately perform spectral matching and identification in reduced time and with reduced computational resources. For example, by attempting to match compressed versions of the unknown spectra to compressed known spectra in the search index, the time required to find a match can be significantly reduced. Furthermore, this enables the match process to be executed with reduced computational hardware requirements. Additionally, the compressed search index reduces the storage and memory requirements related to the matching process as the large spectral libraries are no longer needed locally to perform the matching process. Therefore, this enables the matching process to be executed by devices with reduced storage and memory capabilities in contrast to other methods.

In various instances, machine learning algorithms or models can be implemented in any suitable way to facilitate any suitable aspects described herein. To facilitate some of the above-described machine learning aspects of various embodiments, consider the following discussion of artificial intelligence (AI). Various embodiments described herein can employ artificial intelligence to facilitate automating one or more features or functionalities. The components can employ various AI-based schemes for carrying out various embodiments/examples disclosed herein. In order to provide for or aid in the numerous determinations (e.g., determine, ascertain, infer, calculate, predict, prognose, estimate, derive, forecast, detect, compute) described herein, components described herein can examine the entirety or a subset of the data to which it is granted access and can provide for reasoning about or determine states of the system or environment from a set of observations as captured via events or data. Determinations can be employed to identify a specific context or action, or can generate a probability distribution over states, for example. The determinations can be probabilistic; that is, the computation of a probability distribution over states of interest based on a consideration of data and events. Determinations can also refer to techniques employed for composing higher-level events from a set of events or data.

Such determinations can result in the construction of new events or actions from a set of observed events or stored event data, whether or not the events are correlated in close temporal proximity, and whether the events and data come from one or several event and data sources. Components disclosed herein can employ various classification (explicitly trained (e.g., via training data) as well as implicitly trained (e.g., via observing behavior, preferences, historical information, receiving extrinsic information, and so on)) schemes or systems (e.g., support vector machines, neural networks, expert systems, Bayesian belief networks, fuzzy logic, data fusion engines, and so on) in connection with performing automatic or determined action in connection with the claimed subject matter. Thus, classification schemes or systems can be used to automatically learn and perform a number of functions, actions, or determinations.

A classifier can map an input attribute vector, z = (z1, z2, z3, z4, zn), to a confidence that the input belongs to a class, as by f(z) = confidence(class). Such classification can employ a probabilistic or statistical-based analysis (e.g., factoring into the analysis utilities and costs) to determinate an action to be automatically performed. A support vector machine (SVM) can be an example of a classifier that can be employed. The SVM operates by finding a hyper-surface in the space of possible inputs, where the hyper-surface attempts to split the triggering criteria from the non-triggering events. Intuitively, this makes the classification correct for testing data that is near, but not identical to training data. Other directed and undirected model classification approaches include, e.g., naive Bayes, Bayesian networks, decision trees, neural networks, fuzzy logic models, or probabilistic classification models providing different patterns of independence, any of which can be employed. Classification as used herein also is inclusive of statistical regression that is utilized to develop models of priority.

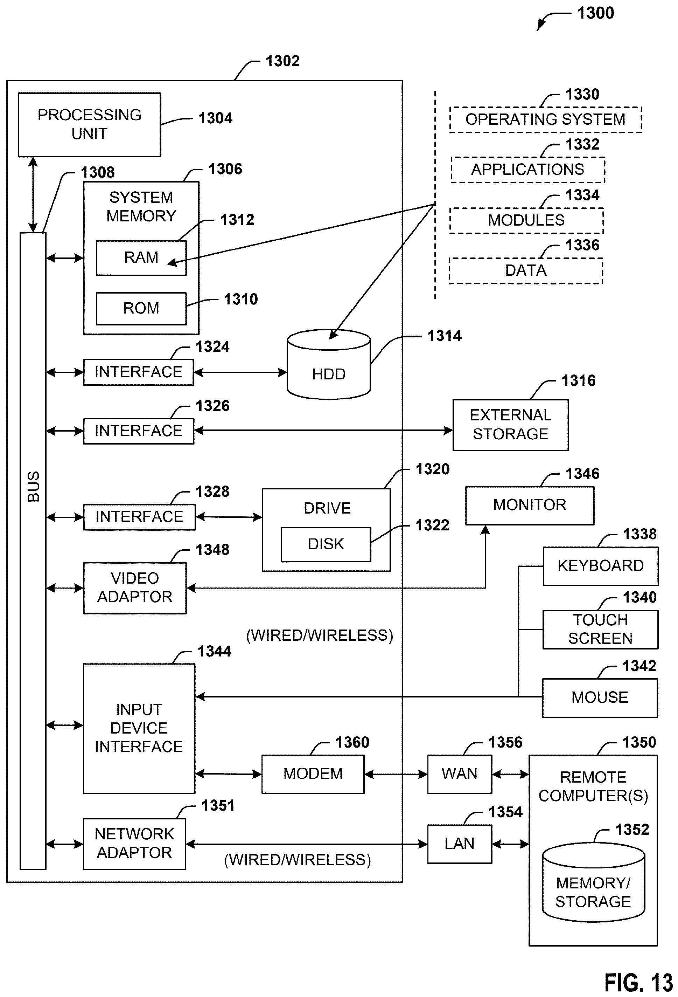

In order to provide additional context for various embodiments described herein, FIG. 13 and the following discussion are intended to provide a brief, general description of a suitable computing environment 1300 in which the various embodiments of the embodiment described herein can be implemented. While the embodiments have been described above in the general context of computer-executable instructions that can run on one or more computers, those skilled in the art will recognize that the embodiments can be also implemented in combination with other program modules or as a combination of hardware and software.

Generally, program modules include routines, programs, components, data structures, etc., that perform particular tasks or implement particular abstract data types. Moreover, those skilled in the art will appreciate that the inventive methods can be practiced with other computer system configurations, including single-processor or multi-processor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can be also practiced in distributed computing environments where certain tasks are performed by remote processing devices that are linked through a communications network. In a distributed computing environment, program modules can be located in both local and remote memory storage devices.

Computing devices typically include various forms of media, including computer-readable storage media, machine-readable storage media, and communication media. Computer-readable or machine-readable storage media include any accessible storage medium, volatile or nonvolatile, removable or non-removable, suitable for storing information such as instructions, program modules, or data.

Computer-readable storage media may include RAM, ROM, EEPROM, flash memory, compact disks (CD-ROM), digital versatile disks (DVD), Blu-ray disks (BD), magnetic cassettes, tapes, disks, solid-state drives, or other tangible or non-transitory media capable of storing desired information. The terms "tangible" or "non-transitory" exclude only propagating transitory signals per se. Such storage media can be accessed by one or more local or remote computing devices for performing read or write operations using standard access protocols.

Communication media include computer-readable instructions, data structures, or program modules transmitted in a modulated data signal, such as a carrier wave, and encompass both wired and wireless transport mechanisms, including acoustic, radio-frequency (RF), and infrared links.

With reference again to FIG. 13, the example environment 1300 includes a computer 1302 configured to execute the spectral data analysis system described herein. The computer 1302 includes a processing unit 1304, a system memory 1306, and a system bus 1308 coupling system components including the system memory 1306 to the processing unit 1304. The processing unit 1304 executes computer-executable instructions implementing the compression component, search component, and library lookup component.

The system memory 1306 and associated storage media store computer-executable instructions and spectral data used by the system. The system memory 1306 may include ROM 1310 and RAM 1312, and may be supplemented with non-volatile storage such as a hard disk drive (HDD) 1314, external storage device 1316, and a drive 1320 for reading from or writing to a disk 1322. The HDD 1314, external storage 1316, and drive 1320 may be connected to the system bus 1308 via interfaces 1324, 1326, and 1328. These storage elements retain spectral libraries, encoded spectra, and trained model parameters for the encoder and decoder.

Program modules stored in the drives and RAM 1312 may include an operating system 1330, a spectral analysis application 1332, other supporting modules 1334, and program data 1336. The operating system 1330 manages execution of the spectral compression and search algorithms implemented by the processor 1304.

The computer 1302 may operate in a networked environment via wired or wireless connections to one or more remote computers 1350, which may include a memory or storage 1352. The network may include a local area network (LAN) 1354, a wide area network (WAN) 1356, or a cloud-based environment. A communication adapter 1358 or modem 1360 enables network connectivity for accessing remote spectral libraries, performing distributed compression operations, or updating trained model parameters.

The external storage interface 1326 can manage storage provided by cloud or network systems as if locally connected, allowing seamless retrieval of known spectra or latent-space data. The computer 1302 may communicate wirelessly with analytical instruments such as an FTIR or Raman device to acquire unknown spectra directly, and output analysis results to a display 1346 connected through a video adapter 1348.

Various non-limiting aspects are described in the following examples.

EXAMPLE 1: A system, comprising: an instrument that captures one or more unknown spectra of a sample; a memory that stores computer executable instructions; and a processor that executes the computer executable instructions stored in the memory, wherein the computer executable instructions comprise: a compression component that compresses the one or more unknown spectra, using an encoder, into one or more compressed unknown spectra in a latent space; and a search component that matches the one or more compressed unknown spectra with one or more compressed known spectra to obtain one or more candidate matches of the sample.

EXAMPLE 2: The system of any preceding example, wherein the compression component compresses the one or more unknown spectra by preprocessing the one or more unknown spectra and passing the preprocessed spectra through the encoder, wherein the preprocessing includes one or more of resampling, scaling, and smoothing.

EXAMPLE 3: The system of any preceding example, wherein the one or more compressed known spectra are generated by compressing one or more spectra of known chemical composition, using the encoder, into the compressed known spectra in the latent space.

EXAMPLE 4: The system of any preceding example, wherein the search component matches the one or more compressed unknown spectra with the one or more compressed known spectra to obtain the one or more candidate matches includes: generating a search tree based on the one or more compressed known spectra; obtaining possible candidate matches by matching the one or more compressed unknown spectra with the one or more compressed known spectra from the search tree; and selecting the candidate matches from the possible candidate matches.

EXAMPLE 5: The system of any preceding example, wherein selecting the candidate matches from the possible candidate matches includes: clustering the compressed known spectra corresponding to the possible candidate matches; and selecting the candidate matches from the possible candidate matches based on the clustering.

EXAMPLE 6: The system of any preceding example, wherein selecting the candidate matches from the possible candidate matches further comprising: performing a regression comparison of the compressed known spectra corresponding to the possible candidate matches; and selecting the candidate matches from the possible candidate matches further based on the regression comparison.

EXAMPLE 7: The system of any preceding example, wherein the compression component further decomposes the unknown spectra to generate component unknown spectra, and wherein compressing the unknown spectra includes compressing the component unknown spectra.

EXAMPLE 8: The system of any preceding example, wherein the computer executable instructions further comprise: a library lookup component that retrieves one or more full spectra corresponding to the one or more candidate matches.

EXAMPLE 9: The system of any preceding example, wherein the search component further calculates one or more residual spectra by removing the one or more compressed known spectra corresponding to the candidate matches from the one or more compressed unknown spectra, and wherein the search component matches the one or more compressed unknown spectra with the one or more compressed known spectra to obtain the one or more candidate matches includes: the search component matches the one or more residual spectra with the one or more compressed known spectra to obtain the one or more candidate matches.

EXAMPLE 10: The system of any preceding example, wherein the library lookup component further determines proportions of the candidate matches in the sample based on a non-negative least squares comparison between the one or more full spectra and the one or more unknown.

EXAMPLE 11: The system of any preceding example, wherein the library lookup component retrieves the one or more full spectra corresponding to the one or more candidate matches by decoding the compressed known spectra corresponding to the candidate matches using a decoder.

EXAMPLE 12: The system of any preceding example, wherein the encoder and the decoder are trained with spectra of known chemical composition.

EXAMPLE 13: The system of any preceding example, wherein the unknown spectra are a series of spectra acquired from the sample, and wherein compressing the one or more unknown spectra includes decomposing the unknown spectra into component unknown spectra and compressing the component unknown spectra to obtain the compressed unknown spectra.

EXAMPLE 14: The system of any preceding example, wherein the computer executable instructions further comprise a training component that trains the encoder, wherein the training component trains the encoder by: augmenting a training dataset comprising one or more spectra of known chemical compositions based on characteristics of an instrument for acquiring the unknown spectra; compressing and decompressing, using the encoder and a decoder, the training dataset; comparing the decompressed training dataset to the training data set; and updating the encoder based on the comparing.

EXAMPLE 15: The system of any preceding example, wherein augmenting the one or more spectra of known chemical composition based on the characteristics of the instrument for acquiring the unknown spectra includes one or more of adding noise, scaling, shifting, and adding instrument specific noise distributions to the spectra of known chemical composition.

In various aspects, any combination or combinations of EXAMPLES 1-15 can be implemented.

EXAMPLE 16: A computer-implemented method, accessing, by a device operatively coupled to a processor, one or more unknown spectra acquired from a sample by an instrument; compressing, by the device, the one or more unknown spectra, using an encoder, into one or more compressed unknown spectra in the latent space; and matching, by the device, the one or more compressed unknown spectra with one or more compressed known spectra to obtain one or more candidate matches of the sample.

EXAMPLE 17: The computer-implemented method of any preceding example, wherein compressing the one or more unknown spectra include preprocessing the one or more unknown spectra and compressing the one or more preprocessed unknown spectra, wherein the preprocessing includes one or more of resampling, scaling and smoothing.

EXAMPLE 18: The computer-implemented method of any preceding example, wherein the matching comprises: generating, by the device, a search tree based on the one or more compressed known spectra; obtaining, by the device, possible candidate matches by matching the one or more compressed unknown spectra with the one or more compressed known spectra from the search tree; and selecting, by the device, the one or more candidate matches from the possible candidate matches.

EXAMPLE 19: The computer-implemented method of any preceding example, wherein selecting the candidate matches from the possible candidate matches includes: clustering, by the device, the compressed known spectra corresponding to the possible candidate matches; and selecting, by the device, the candidate matches from the possible candidate matches based on the clustering.

EXAMPLE 20: The computer-implemented method of any preceding example, further comprising: calculating, by the device, one or more residual spectra by removing the compressed spectra corresponding to the one or more candidate matches from the compressed unknown spectra; and obtaining, by the device, further possible candidate matches by matching the one or more residual spectra with the one or more compressed known spectra.

EXAMPLE 21: The computer-implemented method of any preceding example, wherein compressing the one or more unknown spectra includes decomposing the unknown spectra into component unknown spectra and compressing the component unknown spectra to obtain the compressed unknown spectra.

EXAMPLE 22: The computer-implemented method of any preceding example, wherein the encoder is a trained encoder, and the method further comprises training the encoder by: augmenting, by the device a training dataset comprising one or more known spectra based on characteristics of the instrument; compressing, by the device, and decompressing, using the encoder and a decoder, the training data; comparing, by the device, the decompressed training data to the training data; and updating, by the device, the encoder based on the comparing.

EXAMPLE 23: The computer-implemented method of any preceding example, wherein the instrument is a FTIR system, a Raman system, a mass spectrometry system, or an UV-Vis spectroscopy system.

In various aspects, any combination or combinations of EXAMPLES 16-23 can be implemented.

EXAMPLE 24: A computer program product comprising a non-transitory computer-readable memory having program instructions embodied therewith, the program instructions executable by a processor to cause the processor to: access, by the processor, one or more unknown spectra acquired from a sample by an instrument; compress, by the processor, the one or more unknown spectra, using an encoder, into one or more compressed unknown spectra in a latent space; and match, by the processor, the one or more compressed unknown spectra with one or more compressed known spectra to obtain one or more candidate matches.

EXAMPLE 25: The computer program product of any preceding example, wherein compressing the one or more unknown spectra includes preprocessing the one or more unknown spectra and compressing the one or more preprocessed unknown spectra, wherein the preprocessing includes one or more of resampling, scaling and smoothing.

EXAMPLE 26: The computer program product of any preceding example, wherein the matching comprises: generating, by the processor, a search tree based on the one or more compressed known spectra; obtaining, by the processor, possible candidate matches by matching the one or more compressed unknown spectra with the one or more compressed known spectra from the search tree; and selecting, by the processor, the candidate matches from the possible candidate matches.

EXAMPLE 27: The computer program product of any preceding example, wherein selecting the candidate matches from the possible candidate matches includes: clustering, by the processor, the compressed known spectra corresponding to the possible candidate matches; and selecting, by the processor, the candidate matches from the possible candidate matches based on the clustering.

EXAMPLE 28: The computer program product of any preceding example, further comprises program instructions to cause the processor to: wherein generating possible candidate matches from the index by searching the search tree built from the compressed known spectra includes: calculate, by the processor, one or more compressed residual spectra by removing the compressed spectra corresponding to the one or more candidate matches from the compressed unknown spectra; and obtain, by the processor, further possible candidate matches by matching the one or more compressed residual spectra with the one or more compressed known spectra.

EXAMPLE 29: The computer program product of any preceding example, wherein compressing the one or more unknown spectra includes decomposing the unknown spectra into component unknown spectra and compressing the component unknown spectra to obtain the compressed unknown spectra.

In various aspects, any combination or combinations of EXAMPLES 24-29 can be implemented.

In various aspects, any combination or combinations of EXAMPLES 1-29 can be implemented.

## Claims

1. A system for spectral data analysis, comprising:
an instrument that captures one or more unknown spectra of a sample;
a memory that stores computer executable instructions; and
a processor that executes the computer executable instructions stored in the memory,
wherein the computer executable instructions comprise:
a compression component that compresses the one or more unknown spectra, using an encoder, into one or more compressed unknown spectra in a latent space; and
a search component that matches the one or more compressed unknown spectra with one or more compressed known spectra to obtain one or more candidate matches of the sample.

2. The system of claim 1, wherein the compression component compresses the one or more unknown spectra by preprocessing the one or more unknown spectra and passing the preprocessed spectra through the encoder, wherein the one or more unknown spectra is preprocessed based on a configuration of the instrument and the encoder.

3. The system of claim 1, wherein the one or more compressed known spectra are generated by compressing one or more spectra of known chemical composition, using the encoder, into the compressed known spectra in the latent space.

4. The system of any of claims 1-3, wherein the search component matches the one or more compressed unknown spectra with the one or more compressed known spectra to obtain the one or more candidate matches includes:
obtaining possible candidate matches by matching the one or more compressed unknown spectra with the one or more compressed known spectra; and
selecting the candidate matches from the possible candidate matches.

5. The system of claim 4, wherein selecting the candidate matches from the possible candidate matches includes:
clustering the compressed known spectra corresponding to the possible candidate matches;
performing a regression comparison of the compressed unknown spectra with the compressed known spectra of the possible candidate matches; and
selecting the candidate matches from the possible candidate matches based on the clustering.

6. The system of claim 4, wherein the search component further generates a search tree based on the one or more compressed known spectra, and obtains possible candidate matches by matching the one or more compressed unknown spectra with the one or more compressed known spectra from the search tree.

7. The system of claim 1, wherein the compression component further decomposes the unknown spectra to generate component unknown spectra, and wherein compressing the unknown spectra includes compressing the component unknown spectra.

8. The system of claim 1, wherein the search component further calculates one or more residual spectra, and wherein the search component matches the one or more compressed unknown spectra with the one or more compressed known spectra to obtain the one or more candidate matches includes: the search component matches the one or more residual spectra with the one or more compressed known spectra to obtain the one or more candidate matches.

9. The system of any of claims 1-8, wherein the computer executable instructions further comprise:
a library lookup component that retrieves one or more full spectra corresponding to the one or more candidate matches.

10. The system of claim 9, wherein the library lookup component further determines proportions of the candidate matches in the sample based on a non-negative least squares comparison between the one or more full spectra and the one or more unknown spectra.

11. The system of claim 9, wherein the library lookup component retrieves the one or more full spectra corresponding to the one or more candidate matches by decoding the compressed known spectra corresponding to the candidate matches using a decoder.

12. The system of any of claims 1-11, wherein the unknown spectra are a series of spectra acquired from the sample, and wherein compressing the one or more unknown spectra includes decomposing the unknown spectra into component unknown spectra and compressing the component unknown spectra to obtain the compressed unknown spectra.

13. The system of any of claims 1-12, wherein the computer executable instructions further comprise a training component that trains the encoder, wherein the training component trains the encoder by:
augmenting a training dataset comprising one or more spectra of known chemical compositions based on characteristics of the instrument for acquiring the unknown spectra;
compressing and decompressing, using the encoder and a decoder, the training dataset;
comparing the decompressed training dataset to the training data set; and
updating the encoder based on the comparing.

14. A computer-implemented method for spectral data analysis, comprising:
accessing one or more unknown spectra acquired from a sample by an instrument;
compressing the one or more unknown spectra, using an encoder, into one or more compressed unknown spectra in a latent space; and
matching the one or more compressed unknown spectra with one or more compressed known spectra to obtain one or more candidate matches of the sample.

15. The computer-implemented method of claim 14, further comprising:
accessing one or more second unknown spectra acquired from a second sample by a second instrument, wherein the first instrument and the second instrument have different configurations;
compressing the one or more second unknown spectra, using the encoder, into one or more second compressed unknown spectra; and
matching the one or more second compressed unknown spectra with the one or more compressed known spectra to obtain one or more second candidate matches of the second sample.
